# EUROPÄISCHE PATENTANMELDUNG

(11) **EP 1 930 500 A1**
(43) Veröffentlichungstag der Anmeldung: **11.06.2008**
(21) Anmeldenummer: 06025405.9
(22) Anmeldetag: 08.12.2006
(51) Int. Cl.: D21H 21/24, D21H 27/00, D21H 17/20, A61K 8/00, D06M 15/00

(54) **Emulsionen zur Avivage von Textilien und Papier**

(71) Anmelder: Cognis IP Management GmbH, 40589 Düsseldorf (DE)
(72) Erfinder: Sorns, Jörg, 40476 Düsseldorf (DE); Kawa, Rolf, 40789 Monheim (DE); Eichorn, Stephan, Dr., 64579 Gernsheim (DE); Urban, Andrea, 67069 Ludwigshafen (DE)

(57) **Zusammenfassung**

Gegenstand der Erfindung sind W/O -Emulsionen enthaltend
(a) 12-21 Gew.-% Polygylcerin-2-Dipolyhydroxystearat
(b) 4-7 Gew.-% eines hydrierten ethoxylierten Ricinusöls
(c) 25-35 Gew.% Mineralöl
(d) 20-30 Gew.-% weiterer Ölkörper
(e) 3-5 Gew.-% Hydrotrope
(f) 3-4 Gew.-% Metallseifen und
(g) 10-30 Gew.-% Wasser
bezogen auf die Gesamtzusammensetzung.

## Beschreibung

### Gebiet der Erfindung

Gegenstand der Erfindung sind spezielle Emulsionen, die zur Imprägnierung und Benetzung von Gebrauchs- und Hygienepapiertücher eingesetzt werden können.

### Stand der Technik

Unter dem Oberbegriff "Papier" werden ca. 3000 verschiedene Sorten und Artikel verstanden, deren Beschaffenheit und Anwendungsgebiete sich zum Teil erheblich unterscheiden können. Zur Herstellung von Papier benötigt man eine Reihe von Zusatzstoffen, von denen Füllstoffe (z.B. Kreide oder Kaolin) und Bindemittel (z.B. Stärke) zu den wichtigsten zählen. Für den Bereich der Tissue- und Hygienepapiere, die in engeren Kontakt mit der menschlichen Haut gebracht werden, besteht ein besonderes Bedürfnis nach einem angenehmen Weichgriff, der dem Papier üblicherweise durch eine sorgfältige Auswahl der Faserstoffe und insbesondere einen hohen Anteil an frischem Holzschliff oder Cellulose verliehen wird. Im Hinblick auf die Wirtschaftlichkeit der Papierherstellung sowie aus ökologischer Sicht, ist es jedoch wünschenswert, möglichst hohe Anteile an qualitativ minderwertigerem Altpapier mitzuverwenden. Dies hat jedoch zur Folge, daß der Weichgriff des Papiers signifikant verschlechtert wird, was von den Anwendern als störend empfunden wird und insbesondere bei häufigem Gebrauch auch zu Hautirritationen führen kann.

In der Vergangenheit hat es daher nicht an Versuchen gemangelt, Tissuepapiere durch Tränken, Beschichten oder andere Oberflächenbehandlung so zu modifizieren, daß ein angenehmerer Weichgriff resultiert. Hierfür müssen spezielle Lotionen und Emulsionen entwickelt werde, die sich einerseits leicht auf das Papier aufgetragen lassen, anderseits die Papierstruktur nicht negativ beeinflussen. Um den Weichgriff zu verbessern, werden häufig Niotenside oder eine Kombination aus Nio- und Aniontensiden verwendet. Auch Polysiloxane und kationische Polymere werden für diesen Zweck eingesetzt.

Gegenstand der internationalen Patentanmeldung WO 95/35411 sind Tissuepapiere, die mit Avivagemitteln beschichtet werden, welche 20 bis 80 Gew.-% eines wasserfreien Emolliens (Mineralöle, Fettsäureester, Fettalkoholethoxylate, Fettsäureethoxylate, Fettalkohole und deren Mischungen), 5 bis 95 Gew.-% eines das Emolliens "immobilisierenden Agens" (Fettalkohole, Fettsäuren oder Fettalkoholethoxylate mit jeweils 12 bis 22 Kohlenstoffatomen im Fettrest) sowie 1 bis 50 Gew.-% Tenside mit einem HLB-Wert von vorzugsweise 4 bis 20 enthalten. Die in der Schrift aufgeführten Ausführungsbeispiele enthalten als Emolliens ausnahmslos Petrolatum. Die internationale Patentanmeldung WO 95/35412 offenbart ähnliche Tissuepapiere, wobei als Softener wasserfreie Mischungen von (a) Mineralölen, (b) Fettalkoholen oder Fettsäuren und (c) Fettalkoholethoxylaten zum Einsatz kommen. Gegenstand der intenationalen Patentanmeldung WO 95/16824 sind Avivagemittel für Tissuepapiere, die Mineralöl, Fettalkoholethoxylate und nichtionische Tenside (Sorbitanester, Glucamide) enthalten. Des weiteren werden in der internationalen Patentanmeldung WO 97/30216 (Kaysersberg) flüssige Avivagemittel für Papiertaschentücher auf Basis von langkettigen, gesättigten Fettalkoholen und Wachsestern mit insgesamt wenigstens 24 Kohlenstoffatomen beschrieben, die einen sehr hohen Wasseranteil enthalten. Vom anwendungstechnischen Standpunkt sind Weichgriff, Verarbeitungsverhalten und Sensorik der behandelten Papiere nach wie vor verbesserungswürdig. Gegenstand der DE 10102500 A1 sind Lotionen, die (a) Polyolpoly-12-hydroxystearate, (b) Ölkörper ausgewählt aus der Gruppe der Glyceride, Kohlenwasserstoffe, Silikonöle, Dialkylether und der Dialkylcarbonate, oder beliebigen Gemischen davon und (c) 5-25 Gew.-% Wasser enthalten und sich, wie in der EP 1 225 276 A1 beschrieben, auch zur Beschichtung von Papieren eigne. Diese Emulsionen und Lotionen sind bezüglich der Stabilität noch verbesserungswürdig. Insbesondere lassen sich häufig keine Pflanzenextrakte ohne farbliche Veränderung einarbeiten, was sich nachteilig auf den Weißgrad des zu beschichtenden Papiers auswirkt.

Der Erfindung lag die Aufgabe zugrunde, Emulsionen mit verbesserter Stabilität zur Verfügung zu stellen, mit deren Hilfe man trockene Gebrauchspapiere, insbesondere Tissuepapiere, aber auch Tissue-Gewebe beschichten kann, ohne den Weißgrad des Papiers signifikant herabzusetzen. Die Emulsionen sollten ausgezeichnete pflegende Eigenschaften aufweisen, hinsichtlich sensorischer Eigenschaften denen klassischer Skin-Care-Formulierungen ähneln. Ein weiterer Aspekt der Aufgabe bestand darin, Zubereitungen zur Verfügung zu stellen, die auch mit Tissue-Papieren mit einem hohen Altpapieranteil kompatibel sind. Gleichzeitig sollten nur leicht biologisch abbaubare Hilfsstoffe Verwendung finden und die Zubereitungen leicht in das Tissue eindringen, sich homogen verteilen, einen relativ niedrigen Wassergehalt aufweisen und dennoch in hochkonzentrierter Form eine so niedrige Viskosität aufweisen, daß sie sich leicht verarbeiten lassen.

### Beschreibung der Erfindung

Es wurde nun überraschenderweise gefunden, daß Zubereitungen auf Basis bestimmter Emulgatoren, einem hohen Anteil an Mineralölen und einem definierten Wassergehalt nicht nur einen angenehmen Weichgriff der Papierprodukte gewährleisten, sondern auch stabiler sind als die bekannten Beschichtungslotionen/-emulsionen des Standes der Technik. Selbst bei Einarbeitung von Pflanzenextrakten, die eine Eigenfarbe aufweisen, nimmt der Weißgrad des Papiers bei Beschichtung des Papiers mit den erfindungsgemäßen Lotionen nicht signifikant ab.

Gegenstand der Erfindung sind daher W/O- Emulsionen enthaltend:
(a) 12 - 21 Gew.-% Polygylcerin-2-Dipolyhydroxystearat
(b) 4 - 7 Gew.-% eines hydrierten ethoxylierten Ricinusöls
(c) 25 - 35 Gew.% Mineralöl
(d) 20 - 30 Gew.-% weiterer Ölkörper
(e) 3 - 5 Gew.-% Hydrotrope
(f) 3 - 4 Gew.-% Metallseifen und
(g) 10 - 30 Gew.-% Wasser
bezogen auf die Gesamtzusammensetzung.

Die erfindungsgemäßen Emulsionen sind flüssig, bei 20 °C pumpfähig und daher sehr leicht verarbeitbar. Die Emulsionen weisen aufgrund des hohen Anteils an Ölen gute Pflegeeigenschaften auf und sind deutlich stabiler als vergleichbare Emulsionen des Standes der Technik, die zur Beschichtung verwendet werden. Sie zeichnen sich durch eine geringere Beeinflussung des Weißgrades des zu beschichtenden Papiermaterials aus und erzeugen auf der Haut eine gute sensorische Glätte. Die Komponenten der Emulsion müssen genau aufeinander abgestimmt sein, vorzugsweise enthalten die erfindungsgemäßen Emulsionen keine weiteren Bestandteile außer rohstoffbedingte Nebenprodukte, d.h. die Emulsionen bestehen im wesentlichen aus den Komponenten (a) bis (g) in den angegeben Mengen. Es hat sich gezeigt, dass nur Mischungen in den angegebenen Verhältnissen die Stabilitätsanforderungen erfüllen, in der Lage sind Wirkstoffe mit Eigenfarbe, wie z.B. Herbalia Chamomila, aufzunehmen, ohne zu einer signifikanten Veränderung im Weißgrad des beschichteten Papiers zu führen. Als Komponente (a) ist beispielsweise Dehymuls® PGPH von der Cognis Deutschland GmbH und Co. KG einsetzbar. Erfindungsgemäß besonders bevorzugt sind Emulsionen, die dadurch gekennzeichnet sind, daß bei Beschichtung von Papieren und Vliesen die Abnahme des Weißgrades des Papiers/Vlieses maximal 4%, insbesondere maximal 3,5 % beträgt. Der Weißgrad wird dabei nach der Methode DIN EN 12625-7, Punkt 7.3.2, Farbe (D 65/10°) gemessen, wobei der Weißgrad von nicht beschichtetem/ungetränktem Papier 100% entspricht.

In einer bevorzugten Ausführungsform weisen die Emulsionen bei 23° C eine Viskosität von 500 - 5000 mPa·s auf, wobei ein Bereich von 500 - 3000 mPa·s und insbesondere von 1000-2000 mPa·s bevorzugt ist (Brookfield-RVF, Spindel 5, 10 UpM, 23° C).

Die Emulsionen enthalten einen Wasseranteil von maximal 30 Gew.-% bezogen auf die Gesamtzusammensetzung. Erfindungsgemäß bevorzugt ist ein Wassergehalt von maximal 25 Gew.-% und besonders bevorzugt maximal 20 Gew.-%.. Die Gesamtmenge der Emulgatoren (a) und (b) kann zwischen 16 - 28 Gew.-% bezogen auf die Gesamtzusammensetzung variieren und beträgt vorzugsweise 15 - 25 Gew.%. Erfindungsgemäß bevorzugt ist, daß das Gewichtsverhältnis der Komponenten (a : b) im Bereich (2,5 - 3,5):1 variiert. Als Komponente (b) ist ein Ethylenoxidanlagerungsprodukt von 7 Mol Ethylenoxid pro Mol hydriertes Ricinusöl bevorzugt. Ein derartiges Produkt ist beispielsweise Dehymuls® HRE 7, das von der Cognis Deutschland GmbH & Co KG vermarktet wird.

### Ölkörper: Komponenten (c) und (d)

Die Gesamtmenge der Ölkörper (c) und (d) variiert zwischen 45 und 65 Gew.-%. In einer bevorzugten Ausführungsform beträgt der Anteil des Mineralöls an der Ölphase wenigstens 45 Gew.-%, vorzugsweise wenigstens 50 Gew.-% und besonders bevorzugt wenigstens 55 Gew.-% bezogen auf die Gesamtmenge der Komponenten (c) und (d). Die Ölphase sollte möglichst dünnflüssig sein, d.h. es sind Ölkörper mit Viskositäten von 2-230 mPa·s bevorzugt, insbesondere als Ölkörper (d). Folgende Mineralöle sind als Kompenente (c) geeignet: Weißöl Pharma 40, Paraffinöl dünnflüssig, Paraffinöl dickflüssig, Paraffinum liquidum, Paraffinum perliquidum, Paraffinum subliquidum.

Als weitere Ölkörper (Komponente d) eignen sich beispielsweise die nachstehend genannten Verbindungsklassen geeignet: Guerbetalkohole auf Basis von Fettalkoholen mit 6 bis 18, vorzugsweise 8 bis 10 Kohlenstoffatomen, z.B. 2-Ethylhexanol oder 2-Octyldodecanol; Ester von linearen oder verzweigten, gesättigten oder ungesättigten C₆-C₂₄-Fettsäuren mit linearen oder verzweigten, gesättigten oder ungesättigten C₆-C₂₄-Fettalkoholen. Beispielhaft seien genannt Hexyllaurat, Myristylisostearat, Myristyloleat, Cetylisostearat, Cetyloleat, Stearylisostearat, Stearyloleat, Isostearylmyristat, Isostearylpalmitat, I-sostearylstearat, Isostearylisostearat, Isostearyloleat, Oleylmyristat, Oleylisostearat, Oleyloleat, Oleylerucat, Erucylisostearat, Erucyloleat, Cococaprylat/caprat. Weitere geeignete Ester sind z.B. Ester von C₁₈-C₃₈-Alkylhydroxycarbonsäuren mit linearen oder verzweigten, gesättigten oder ungesättigten C₆-C₂₂-Fettalkoholen, Ester von linearen und/oder verzweigten, gesättigten oder ungesättigten Fettsäuren mit mehrwertigen Alkoholen (wie z. B. Propylenglycol, Dimerdiol oder Trimertriol) und/oder Guerbetalkoholen, Triglyceride oder Triglyceridmischungen, flüssige Mono-/Di-/Triglyceridmischungen, Ester von C₆-C₂₂-Fettalkoholen und/oder Guerbetalkoholen mit aromatischen Carbonsäuren, insbesondere Benzoesäure (z. B. Finsolv® TN), Ester von C₂-C₁₂-Dicarbonsäuren mit linearen oder verzweigten, gesättigten oder ungesättigten Alkoholen mit 1 bis 22 Kohlenstoffatomen oder Polyolen mit 2 bis 10 Kohlenstoffatomen und 2 bis 6 Hydroxylgruppen. Geeignet sind auch pflanzliche Öle, Triglyceridmischungen, substituierte Cyclohexane, lineare symmetrische oder unsymmetrische Dialkylcarbonate (z.B. Cetiol® CC), Guerbetcarbonate auf Basis von Fettalkoholen mit 6 bis 18, vorzugsweise 8 bis 10 C-Atomen, lineare oder verzweigte, symmetrische oder unsymmetrische Dialkylether mit 6 bis 22 Kohlenstoffatomen pro Alkylgruppe, wie z. B. Di-n-octyl Ether (Cetiol® OE), Ringöffnungsprodukte von epoxidierten Fettsäureestern mit Polyolen, Kohlenwasserstoffe wie Paraffin- oder Mineralöle, Oligo- oder Polyalphaolefine. Die Dialkylcarbonate und Dialkylether können symmetrisch oder unsymmetrisch, verzweigt oder unverzweigt, gesättigt oder ungesättigt sein und lassen sich nach Reaktionen, die aus dem Stand der Technik hinlänglich bekannt sind, herstellen. Geeignete Siliconverbindungen sind beispielsweise Dimethylpolysiloxane, Methylphenylpolysiloxane, cyclische Silicone (Cyclomethicon) sowie amino-, fettsäure-, alkohol-, polyether-, epoxy-, fluor-, glykosid- und/oder alkylmodifizierte Siliconverbindungen. Weiterhin geeignet sind Simethicone.

Bevorzugt geeignet als Komponente (d) sind insbesondere Öle mit Spreitwerten von wenigstens 250mm²/10 min **(**U.Zeidler: Über das Spreiten von Lipiden auf der Haut, Fette-Seifen-Anstrichmittel Nr. 10, 403 - 408, 1985**)**, insbesondere Esteröle, Dialkylether, Guerbetalkohol oder Glyceride mit oben genanntem Spreitwert. Hierzu zählen z.B. Dibutyladipat, Isopropylpalmitat, Hexyllaurat, Ethylhexylstearat, Dicaprylylether, Dicaprylylcarbonate, Hexyldecylstearat, Oleyloleat, Oleylerucat oder Pflanzenöle.

### Hydrotrope (e)

Die erfindungsgemäßen Emulsionen enthalten als Komponente (e) wenigstens ein Hydrotrop, das zur Verbesserung der sensorischen Eigenschaften und Stabilität der Zusammensetzung beiträgt und lösungsvermittelnde Eigenschaften hat. Es dient auch dazu, den Feuchtigkeitsgehalt der Emulsion im Papier stabil zu halten.

Erfindungsgemäß geeignet sind flüssige Polyole und Polyolderivate, z. B. Glycerin, Diglycerin, Triglycerin, Ethylenglycol, Propylenglycol, Butylenglycol, 1,2,6-Hexantriol, sowie flüssige Polyethylenglycole. Erfindungsgemäß bevorzugt geeignet als Feuchthaltemittel sind Glycerin, Diglycerin und Triglycerin. Das Hydrotrop ist in Mengen von 3 - 5 Gew.-%, insbesondere 3,5 - 4,5 Gew.-% bezogen auf die Gesamtzusammensetzung enthalten. Erfindungsgemäß bevorzugt sind Glycerin, Butylenglykol oder Propylenglykol und unter diesen Glycerin.

### Metallseifen (f)

Unter dem Begriff "Metallseifen" versteht der Fachmann wenig wasserlösliche Metallsalze von Fettsäuren (vgl. Römpps Chemielexikon). Sie tragen aufgrund ihrer ölverdickenden Eigenschaften synergistisch zur Stabilität der Emulsion bei. Üblicherweise geeignet sind Calcium-, Magnesium-, Aluminium-, und Zinksalze von C12-C24-Fettsäuren oder C12-C24-Hydroxyfettsäuren. Erfindungsgemäß bevorzugt als Metallseifen sind Magnesiumstearat und/ oder Aluminiumstearat. Letztere sind aufgrund ihrer Strukturausbildung besonders geeignet zur Verdickung und Stabilisierung der Ölphase.

### Reizlindernde/entzündunashemmende Wirkstoffe

Eine bevorzugte Ausführungsform der erfindungsgemäßen Emulsion enthält wenigstens einen reizlindernden/entzündunsghemmenden Wirkstoff, der insbesondere zur Linderung entzündlicher Hautprozesse oder geröteter, wunder Haut dient. Der reizlindernde Wirkstoff ist üblicherweise in einer Menge von 0,01 - 10 Gew.-%, vorzugsweise 0,05 - 5 Gew.-% und insbesondere 0,05 - 3 Gew.-% enthalten.

Erfindungsgemäß bevorzugt sind insbesondere Bisabolol, Allantoin und Panthenol und Bisabolol. Auch Vitamine und Vitaminvorstufen sowie Proteinhydrolysate können die Wundheilung fördern.

Geeignet sind auch Pflanzenextrakte, die häufig eine synergistisch wirkende Kombination wundheilender/reizlindernder Stoffe enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Kamille, Aloe Vera, Hamamelis, Lindenblüten, Salbei, und Melisse geeignet.

### Beispiel

Zwei Rezepturen, eine erfindungsgemäße (1) und eine Vergleichsrezeptur (V1) wurden bezüglich Stabilität und Weißgrad bewertet. Der Weißgrad wurde nach Methode DIN EN 12625-7, Punkt 7.3.2, Farbe(D65/10°) gemessen, wobei der Weißgrad von unbeschichtetem/ungetränktem Papier mit 100% bewertet wurde. Bewertet wurde die Abnahme des Weißgrades in % wobei die Abnahme maximal 4 % betragen durfte. Die Sensorik wurde von geschulten Personen nach dem Prinzip der Schulnoten von 1-6 bewertet.

| **Inhaltsstoffe** | **1** | **V1** |
|---|---|---|
| Polyglycerin-2-dipolyhydroxystearat | 15 | 20,6 |
| Hydriertes Ricinusöl + 7EO | 5 | - |
| Mineralöl | 30 | - |
| Ethylhexyl Stearat | 22,4 | - |
| Cocoglycerid | - | 20,6 |
| Dicaprylyl Ether | - | 20,6 |
| Sorbitan Sesquiolat | - | 4,8 |
| Bienenwachs | - | 3,35 |
| Dicocoyl Pentaerythrityl Disteatyl Citrat | - | 2 |
| Tocopherol und hydriertes Palmglyceridzitrat | - | 0,02 |
| Glycerin | 4 | 7 |
| Magnesium Stearat | 3,5 | - |
| Aluminium Stearat | - | 2,75 |
| Chamomilla recutita (matricaria) Blütenextrakt | 0,1 | 0,1 |
| Wasser | 20 | 18,2 |
| Konservierung | Nach Belieben | |
| | | |
| Aussehen | Milchig-weiß | Milchig-gelb |
| | | |
| Stabilität 4 Wochen - RT | + | + |
| Stabilität 8 Wochen - RT | + | - |
| Stabilität 4 Wochen - 30°C | + | - |
| | | |
| Abnahme des Weißgrades in % | 3,4 | 3,9 |
| | | |
| Sensorik der Lotion - Cremigkeit | 1,0 | 4,0 |

## Patentansprüche

1. W/O -Emulsion enthaltend
(a) 12 - 21 Gew.-% Polygylcerin-2-Dipolyhydroxystearat
(b) 4 - 7 Gew.-% eines hydrierten ethoxylierten Ricinusöls
(c) 25 - 35 Gew.% Mineralöl
(d) 20 - 30 Gew.-% weiterer Ölkörper
(e) 3 - 5 Gew.-% Hydrotrope
(f) 3 - 4 Gew.-% Metallseifen und
(g) 10-30 Gew.-% Wasser
bezogen auf die Gesamtzusammensetzung.

2. Emulsion gemäß Anspruch 1, **dadurch gekennzeichnet, daß** als Komponente (b) ein Ethylenoxidanlagerungsprodukt von 7 Mol Ethylenoxid pro Mol hydriertes Ricinusöl enthalten ist.

3. Emulsion gemäß wenigstens einem der Ansprüche 1 bis 2, **dadurch gekennzeichnet, daß** die Gesamtmenge der Emulgatoren (a) und (b) 15 - 25 Gew.-% bezogen auf die Gesamtzusammensetzung beträgt.

4. Emulsion gemäß wenigstens einem der Ansprüche 1 bis 3, **dadurch gekennzeichnet, daß** das Gewichtsverhältnis der Komponenten (a : b) im Bereich (2,5 - 3,5):1 variiert.

5. Emulsion gemäß wenigstens einem der Ansprüche 1 bis 4, **dadurch gekennzeichnet, daß** das Hydrotrop (e) ausgewählt ist aus Glycerin, Butylenglykol oder Propylenglykol.

6. Emulsion gemäß wenigstens einem der einem der Ansprüche 1 bis 5, **dadurch gekennzeichnet, daß** als Metallseife Magnesiumstearat und/ oder Aluminiumstearat ist.

7. Emulsion gemäß einem der Ansprüche 1 bis 6, **dadurch gekennzeichnet, daß** zusätzlich wenigstens ein reizlindemder Wirkstoff enthalten ist.

8. Emulsion gemäß einem der Ansprüche 1 bis 7, **dadurch gekennzeichnet, daß** sie bei 23° C eine Viskosität von 500 - 5000 mPa·s, vorzugsweise von 500 - 3000 mPa·s und insbesondere von 1000-2000 mPa·s aufweist.

9. Emulsion gemäß einem der Ansprüche 1 bis 8, **dadurch gekennzeichnet, daß** bei Beschichtung von Papieren und Vliesen die Abnahme des Weißgrades des Papiers/Vlieses maximal 4 % beträgt.
